# FASCICULE DE BREVET EUROPEEN

(11) **EP 1 556 380 B1**
(45) Date de publication et mention de la délivrance du brevet: **12.04.2006**
(21) Numéro de dépôt: 03767851.3
(22) Date de dépôt: 21.10.2003
(51) Int. Cl.: C07D 471/04, A61K 31/444, A61K 31/437, A61P 35/00

(54) **DERIVES DE PYRIDOINDOLONE SUBSTITUES EN -3 PAR UN GROUPE HETEROCYCLIQUE, LEUR PREPARATION ET LEUR APPLICATION EN THERAPEUTIQUE**
IN 3-POSITION HETEROCYCLISCH SUBSTITUIERTE PYRIDOINDOLONDERIVATE, IHRE HERSTELLUNG UND IHRE THERAPEUTISCHE VERWENDUNG
PYRIDOINDOLONE DERIVATIVES SUBSTITUTED IN THE 3-POSITION BY A HETEROCYCLIC GROUP, PREPARATION THEREOF AND APPLICATION OF SAME IN THERAPEUTICS

(30) Priorité: 23.10.2002 FR 0213270
(43) Date de publication de la demande: 27.07.2005
(73) Titulaire: Sanofi-Aventis, 75013 Paris (FR)
(72) Inventeur: BOURRIE, Bernard, F-34980 Saint-Gély-du-Fesc (FR); CASELLAS, Pierre, F-34090 Montpellier (FR); DEROCQ, Jean-Marie, F-34570 Murviel les Montpellier (FR); JEGHAM, Samir, FR-34980 Montferrier-Sur-Lez (FR); MUNEAUX, Yvette, F-34270 Les Matelles (FR)
(74) Mandataire: Kugel, Dominique
(86) Numéro de dépôt international: PCT/FR2003/003111
(87) Numéro de publication internationale: WO 2004/037821

(56) Documents cités:
- FR-A- 2 765 581
- US-A- 4 263 304
- TAT'YANA V GOLOVKO ET AL: "A NEW APPROACH TO THE SYNTHESIS OF FUNCTIONALLY-SUBSTITUTED PYRIDO[2,3-D]INDOLES" MENDELEEV COMMUNICATIONS, CAMBRIDGE, GB, vol. 6, 1995, pages 226-227, XP009008999
- FURIHATA C ET AL: "IN VIVO SHORT-TERM ASSAYS FOR TUMOR INITIATION AND PROMOTION IN THE GRANDULAR STOMACH OF FISCHER RATS" MUTATION RESEARCH, AMSTERDAM, NL, vol. 339, no. 1, 1995, pages 15-35, XP009008997 ISSN: 0027-5107
- MOLINA P ET AL: "ANNULATION OF PYRIDINE TO INDOLE BY A TANDEM AZA-WITTIG/ELECTROCYCLIZATION STRATEGY: SYNTHESIS OF PYRIDO[2,3-B]INDOLES" SYNTHESIS, GEORG THIEME VERLAG. STUTTGART, DE, vol. 11, novembre 1989 (1989-11), pages 878-880, XP001146859 ISSN: 0039-7881
- FURIHATA C ET AL: "UNSCHEDULED DNA SYNTHESIS IN RAT STOMACH-SHORT-TERM ASSAY OF POTENTIAL STOMACH CARCINOGENS" BANBURY REPORT, COLD SPRING HARBOR LABORATORY, COLD SPRING HARBOR, US, vol. 13, 1982, pages 123-135, XP009008981 ISSN: 0198-0068

## Description

La présente invention se rapporte à des dérivés de pyridoindolone substitués en -3 par un groupe hétérocyclique, à leur préparation et à leur application en thérapeutique.

Le brevet français n° 97 08409 décrit des composés de formule : 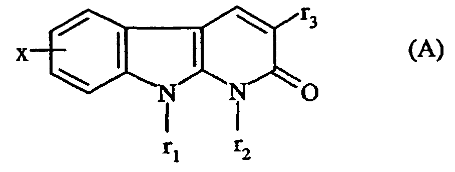 dans laquelle :
- x représente un atome d'hydrogène ou de chlore ou un groupe méthyle ou méthoxy;
- r₁ représente un atome d'hydrogène ou un groupe méthyle ou éthyle ;
- r₂ représente un groupe méthyle ou éthyle ; ou bien
- r₁ et r₂ forment ensemble un groupe (CH₂)₃ ;
- r₃ représente, soit un groupe phényle éventuellement substitué par un atome d'halogène ou un groupe méthyle ou méthoxy, soit un groupe thiényle.

Dans la description de ce brevet, il est mentionné que les composés de formule (A) ayant une affinité pour les sites modulateurs oméga associés aux récepteurs GABA_{A}, peuvent être utilisés dans le traitement d'affections liés aux désordres de la transmission gabaergique associés aux sous-types de récepteurs GABA_{A}, tels que l'anxiété, les troubles du sommeil, l'épilepsie etc...

La présente invention a pour objet des composés ayant une activité anticancéreuse, répondant à la formule : 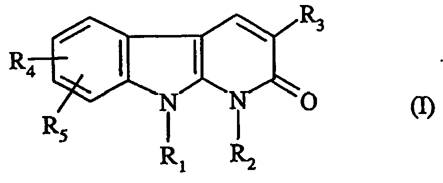 dans laquelle :
- R₁ représente un atome d'hydrogène ou un groupe (C₁-C₄)alkyle ;
- R₂ représente un atome d'hydrogène ou un groupe (C₁-C₄)alkyle ;
- R₃ représente un thiényle mono ou poly substitué par un groupe méthyle ; ou un radical hétérocyclique monocyclique ou bicyclique choisi parmi : un pyridyle, un N-oxydopyridyle, un pyrazole, un (N-phényl)pyrazole, un (N-halogénophényl)pyrazole, un furyle, un indolyle, un (N-benzyl)indolyle, un (N-halogénobenzyl)indolyle, un benzothiényle, un benzofuryle, lesdits radicaux étant non substitués ou substitués une ou plusieurs fois par un atome d'halogène ou un groupe méthyle ou méthoxy ;
- R₄ et R₅ sont identiques ou différents et représentent chacun indépendamment un atome d'hydrogène ou d'halogène ou un groupe hydroxyle, un hydroxyméthyle, (C₁-C₄)alkyle, trifluorométhyle, (C₁-C₄)alcoxy, (C₁-C₄)alcoxycarbonyle, un cyano.

Les composés de formule (I) peuvent exister à l'état de bases ou de sels d'addition à des acides. De tels sels d'addition font partie de l'invention.

Ces sels sont avantageusement préparés avec des acides pharmaceutiquement acceptables, mais les sels d'autres acides utiles, par exemple, pour la purification ou l'isolement des composés de formule (I) font également partie de l'invention.

Les composés de formule (I) peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.

Dans le cadre de la présente invention, on entend par :
- un atome d'halogène : un fluor, un chlore, un brome, ou un iode ;
- un groupe (C₁-C₄)alkyle : un groupe aliphatique saturé linéaire ou ramifié comportant 1 à 4 atomes de carbone. A titre d'exemple, on peut citer les groupes méthyle, éthyle, propyle, isopropyle, butyle, isobutyle, *tert*-butyle ;
- un groupe (C₁-C₄)alcoxy : un radical O-alkyle où le groupe alkyle est tel que précédemment défini.

L'invention a tout particulièrement pour objet les composés de formule (I) dans laquelle :
- R₁ représente un atome d'hydrogène ou un groupe (C₁-C₄)alkyle ;
- R₂ représente un atome d'hydrogène ou un groupe (C₁-C₄)alkyle ;
- R₃ représente un thiényle mono ou poly substitué par un groupe méthyle ; ou un radical hétérocyclique monocyclique ou bicyclique choisi parmi : un pyridyle, un pyrazole, un (N-phényl)pyrazole, un (N-halogénophényl)pyrazole, un furyle, un indolyle, un (N-benzyl)indolyle, un (N-halogénobenzyl)indolyle, un benzothiényle, un benzofuryle, lesdits radicaux étant non substitués ou substitués une ou plusieurs fois par un atome d'halogène ou un groupe méthyle ;
- R₄ et R₅ sont identiques ou différents et représentent chacun indépendamment un atome d'hydrogène ou d'halogène ou un groupe hydroxyle, un hydroxyméthyle, (C₁-C₄)alkyle, trifluorométhyle, (C₁-C₄)alcoxy, (C₁-C₄)alcoxycarbonyle, un cyano.

Parmi les composés de formule (I) objets de l'invention, on peut citer les composés préférés qui se définissent comme suit :
- R₁ représente un atome d'hydrogène ou un groupe méthyle ;
- et/ou R₂ représente un groupe méthyle ;
- et/ou R₃ représente un radical hétérocyclique choisi parmi un N-oxidopyridyle, un pyridyle, un benzothiényle, lesdits radicaux étant non substitués ou substitués une
ou plusieurs fois par un atome d'halogène ou un groupe méthyle ou méthoxy ;
- et/ou R₄ représente un groupe méthyle ;
- et/ou R₅ représente un atome d'hydrogène ou un groupe méthyle.

La présente invention a particulièrement pour objet les composés suivants :
- 1,6-Diméthyl-3-pyridin-4-yl-1,9-dihydro-2*H*-pyrido[2,3-b]indol-2-one.
- 1,6,9-Triméthyl-3-pyridin-4-yl-1,9-dihydro-2*H*-pyrido[2,3-b]indol-2-one.
- 1,6-Diméthyl-3-(1-oxidopyridin-4-yl)-1,9-dihydro-2*H*-pyrido[2,3-b] indol-2-one.
- 3-(4,6-Dimëthylpyridin-2-yl)-1,6-diméthyl-1,9-dihydro-2*H*-pyrido[2,3-b] indol-2-one.
- 3-(1-Benzothien-5-yl)-1,6-diméthyl-1,9-dihydro-2*H*-pyrido[2,3-b] indol-2-one.
- 3-(5-Chloro-1-benzothién-3-yl)-1,6,9-triméthyl-1,9-dihydro-2*H*-pyrido[2,3-b]indol-2-one.
- 3-(1*H*-Indol-1-yl)-1,6-diméthyl-1,9-dihydro-2*H*-pyrido[2,3-b] indol-2-one.
- 3-(3-Chloropyridin-4-yl)-1,6-diméthyl-1,9-dihydro-2*H*-pyrido[2,3-b] indol-2-one.
- 3-(2,6-Diméthoxypyridin-4-yl)-1,6-diméthyl-1,9-dihydro-2*H*-pyrido[2,3-b] indol-2-one.
- 3-(2,6-Diméthoxypyridin-4-yl)-1,6,9-triméthyl-1,9-dihydro-2*H*-pyrido[2,3-b] indol-2-one.
ainsi que leurs sels, hydrates et solvats.

Conformément à l'invention, on peut préparer les composés de formule générale (I) selon le procédé qui suit.

Ce procédé est caractérisé en ce que :
on fait réagir un 2-aminoindole de formule : 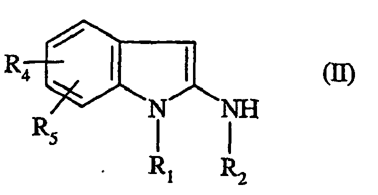
dans laquelle R₁, R₂, R₄ et R₅ sont tels que définis pour un composé de formule (I) avec un ester de formule : 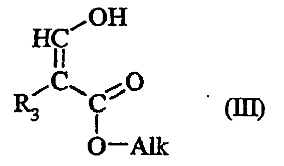 dans laquelle R₃ est tel que défini pour un composé de formule (I) et Alk représente un alkyle en C₁-C₄.

La réaction est effectuée dans un solvant aprotique, polaire et de préférence basique, par exemple dans la pyridine, à une température comprise entre la température ambiante et la température de reflux du solvant.

Les aminoindoles de formule (II) peuvent être préparés par des méthodes telles que celles décrites dans Khim. Geterosikl. Soedin., 1973, 12, 647-652 et dans J. Heterocycl. Chem., 1975, 12, 135-138.

Certains dérivés de 2-aminoindole de formule (II) sont connus et décrits dans Khim. Geterotsikl. Soedin., 1973, 4, 511-515 ; Eur. J. Med. Chem. Chim. Ther., 1992, 27 (9), 908-918 ; Chem. Heterocycl. Compd. (Engl. Transl.), 1970, 6, 338-343 ; Tetrahedron, 1971, 27, 775-785 ; Pharm. Chem. J. (Engl. Transl.), 1990, 24 (11), 810-812 ; Tetrahedron Lett., 1996, 37 (28), 4931-4932.

Les esters de formule (III) peuvent être préparés selon le schéma réactionnel suivant :

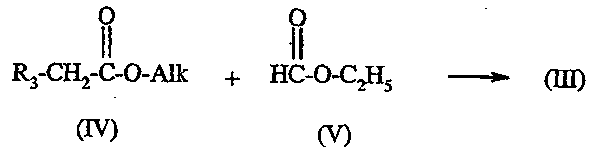

Les esters acétiques de formule (IV) ou les dérivés acide correspondants sont généralement connus et/ou commerciaux.

L'ester méthylique de l'acide 2-(4,5-diméthyl-2-thiényl)acétate est décrit dans J. Heterocycl. Chem., 1988, 25, 1571-1581.

On peut également préparer les composés selon l'invention par un procédé caractérisé en ce que :
on fait réagir un aminoindole de formule : 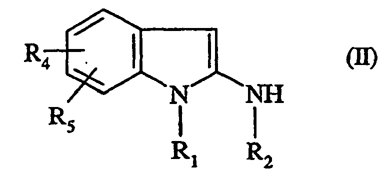
dans laquelle R₁, R₂, R₄ et R₅ sont tels que définis pour un composé de formule (I) avec un ester de formule : 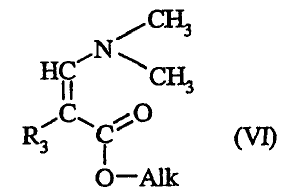 dans laquelle R₃ est tel que défini pour un composé de formule (I) et Alk représente un alkyle en C₁-C₄.

La réaction est effectuée dans un solvant protique et polaire, de préférence en milieu acide, à une température comprise entre la température ambiante et la température de reflux du solvant.

La préparation du composé de formule (VI) est effectuée au moyen du diméthoxy-N,N-diméthylméthanamine (VII) d'après une méthode similaire à celle décrite dans J. Org. Chem., 1982, 47, 2846-2851 ou en utilisant le réactif de Bredereck (*tert*butoxybis(diméthylamino)méthane) selon J. Org. Chem., 1982, 15, 2846-2851 et selon le schéma réactionnel ci-après :

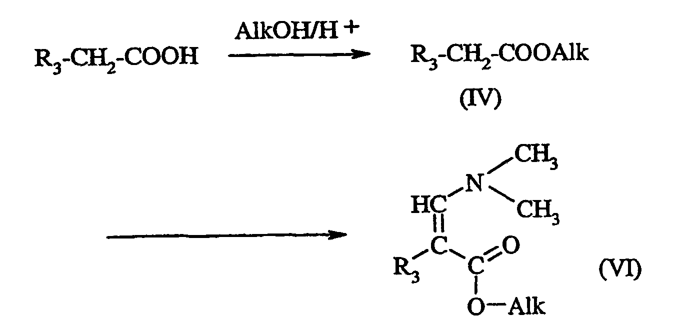

Les exemples suivants décrivent la préparation de certains composés conformes à l'invention. Ces exemples ne sont pas limitatifs et ne font qu'illustrer la présente invention. Les numéros des composés exemplifiés renvoient à ceux donnés dans le tableau ci-après, qui illustre les structures chimiques et les propriétés physiques de quelques composés selon l'invention.

Les signaux observés en résonance magnétique nucléaire (RMN) du proton sont enregistrés dans le DMSO-d₆ contenant éventuellement du TFA, la référence est placée dans le DMSO qui se situe à 2,50 ppm du tétréméthylsilane. Les déplacements chimiques δ sont exprimés en ppm, les signaux sont exprimés ainsi : s: singulet ; se :
singulet élargi ; d : doublet ; d.d : doublet dédoublé ; t : triplet ; td : triplet dédoublé ; q : quadruplet ; m : massif ; mt : multiplet.

Dans les préparations et exemples qui vont suivre, les abréviations suivantes sont utilisées :
TFA : acide trifluoroacétique
DMSO : diméthylsulfoxyde
DCM : dichlorométhane
DMF : diméthylformamide
AcOEt : acétate d'éthyle
AcOH : acide acétique
MTBE : méthyl *tert*-butyl éther
TA : température ambiante
CCM : chromatographie sur couche mince.

### Préparation des composés de formule (II).

Les composés de formule (II) peuvent exister sous 2 formes tautomères :

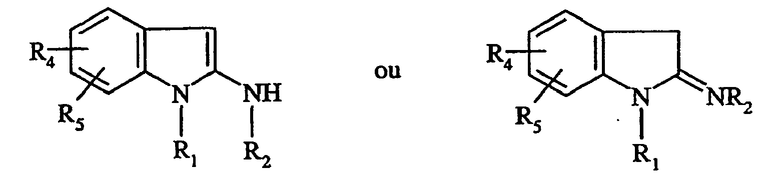

### Préparation 1.1

### Chlorhydrate de N,1,5-triméthyl-1H-indole-2-amine.

### A) N'-(4-méthylphényl)acétohydrazide.

104,8 g de chlorhydrate de 1-(4-méthylphényl)hydrazine sont mis en suspension dans 525 ml d'acétate d'isopropyle, on ajoute une solution de 104,8 g de carbonate de potassium dans 300 ml d'eau puis on agite jusqu'à disparition du solide. Tout en maintenant la température inférieure à 20°C, on ajoute 77,4 g d'anhydride acétique puis on laisse sous agitation à 20°C, on observe la formation d'un précipité qui disparaît lorsque l'on chauffe vers 55-60°C. On lave la phase organique 2 fois par 200 ml d'eau puis on refroidit à 0-5°C pendant une nuit. On récupère le produit formé par filtration puis on le lave 2 fois par 100 ml de MTBE.

RMN CDCl₃ (300 MHz) : 2,02 ppm : s : 3H ; 2,29 ppm : s : 3H ; 6,14 ppm : d : 1H ; 6,73 ppm : d : 2H ; 7,03 ppm : d : 2H ; 7,72 ppm : s : 1H.

### B) N,N'-diméthyl-N'-(4-méthylphényl)acétohydrazine.

On met en suspension 60 g d'hydrazine de l'étape précédente et 11,8 g de bromure de tétrabutylammonium dans 240 ml de toluène et on ajoute 292 g de NaOH à 50 % dans de l'eau puis 155,6 g d'iodure de méthyle. On ajoute alors 83 g de soude en pastilles puis on chauffe le milieu réactionnel à 80°C pendant 6 heures. On refroidit vers 30-35°C puis on ajoute 500 ml d'eau. La phase organique est lavée 3 fois par 100 ml d'eau. La phase organique est séchée par distillation azétropique de l'eau sous pression réduite.

RMN CDCl₃ (300 MHz) : 2,15 ppm : s : 3H ; 2,31 ppm : s : 3H ; 2,95 ppm : s : 3H ; 3,10 ppm : s : 3H ; 6,63 ppm : d : 2H ; 7,13 ppm : d : 2H.

### C) Chlorhydrate de N,1,5-triméthyl-1H-indole-2-amine.

Le produit obtenu à l'étape précédente est dissous dans du toluène et l'on ajoute 61,5 g d'oxychlorure de phosphore et on chauffe à 80°C pendant 2 heures. On ajoute 100 ml d'acétate d'éthyle à 80°C puis on refroidit le milieu à TA. Le précipité est filtré puis lavé 2 fois par 50 ml d'acétate d'éthyle, F = 222°C.

RMN DMSO-d₆ (200 MHz) : 2,36 ppm : s : 3H ; 3,11 ppm : s : 3H ; 3,49 ppm : s : 3H ; 4,29 ppm : s : 1H ; 7,25-7,35 ppm : m : 3H ; 10,07 ppm : m : 1H.

### Préparation 1.2

### Dichlorhydrate de N,5-diméthyl-1H-indole-2-amine.

### A) N'-(4-méthylphényl)acétohydrazide.

Un autre procédé de préparation de ce composé est décrit ci-après.

On dissout 5 g de chlorhydrate de 1-(4-méthylphényl)hydrazine dans de l'eau puis on ajoute de la triéthylamine jusqu'à neutralisation du sel. On extrait à l'AcOEt puis on évapore à sec. On dissout le précipité formé dans 30 ml d'éther puis on ajoute, goutte à goutte, une solution de 4,6 ml d'anhydride acétique dissous
dans 30 ml d'éther. On agite 15 minutes à 0°C puis on filtre le précipité formé pour obtenir 3 g du composé attendu.

RMN CDCl₃ (300 MHz) : 2,02 ppm : s : 3H ; 2,29 ppm : s : 3H ; 6,14 ppm : d : 1H ; 6,73 ppm : d : 2H ; 7,03 ppm : d : 2H ; 7,72 ppm : s : 1H.

### B) N-Méthyl-N'-(4-méthylphényl)acétohydrazide.

On met en suspension 0,8 g de NaH à 60 % dans 30 ml de DMF. On ajoute à 0°C, goutte à goutte, 3,2 g d'hydrazine obtenu à l'étape précédente dans 20 ml de DMF. Lorsqu'il n'y a plus de dégagement gazeux, on ajoute 1,8 ml d'iodure de méthyle et on agite 1 heure à TA. On verse sur une solution de NH₄Cl saturée puis on extrait à l'AcOEt. On lave plusieurs fois avec une solution de NaCl saturée puis on évapore à sec. On purifie par chromatographie sur colonne de silice éluée avec un mélange AcOEt/heptane (25/75 ; v/v) puis (50/50 ; v/v) pour obtenir 1,0 g du composé attendu sous forme de poudre blanche.

RMN CDCl₃ (200 MHz) : 2,21 ppm : s : 3H ; 2,32 ppm : s : 3H ; 3,15 ppm : s : 3H ; 5,88 ppm : s : 1H ; 6,64 ppm : d : 2H ; 7,12 ppm : d : 2H.

### C) Dichlorhydrate de N,5-diméthyl-1H-indole-2-amine.

On dissout 1,0 g du composé de l'étape précédente dans 20 ml de POCl₃ puis on chauffe à 100°C pendant 2 heures. On refroidit le mélange réactionnel puis on ajoute de l'éther. On filtre le précipité formé et on le lave à l'éther pour obtenir 1,3 g du composé attendu.

RMN DMSO-d₆ (300 MHz) : 2,31 ppm : s : 3H ; 3,05 ppm : s : 3H ; 4,14 ppm : s : 2H ; 7,07-7,23 ppm : m : 3H ; 10,51 ppm : s : 1H ; 12,37 ppm : d : 1H.

### Préparation des intermédiaires de formule (III) et (VI).

Les composés (IIn peuvent exister sous 2 formes tautomères :

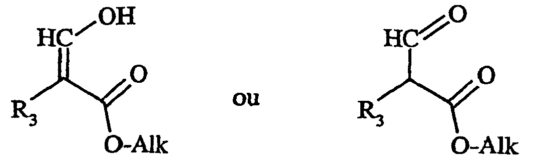

### Préparation 2.1

### 3-(Diméthylamino)-2-(2-pyridinyl)-2-propénoate de méthyle (VI).

On mélange 10 g de 2-(2-pyridinyl)acétate d'éthyle, 39 ml de diméthoxy-N,N-diméthylméthanamine et 1,5 ml de N,N,N',N'-tétraméthylènediamine et on chauffe à 130°C pendant 18 heures. On reprend par 120 ml d'un mélange équivoiumique d'AcOEt et d'une solution saturée de NH₄Cl. On décante la phase organique, on la lave par une solution de NaCl saturée puis on sèche sur Na₂SO₄ et évapore pour obtenir le produit attendu sous forme d'huile.

RMN (250 MHz DMSO) : 1,1 ppm : t : 3H ; 2,6 ppm : s : 6H ; 4 ppm : q : 2H ; 7,1-7,3 ppm : m : 2H . 7,5 ppm : s : 1H ; 7,6 ppm : mt : 1H ; 8,5 ppm : d : 1H.

### Préparation 2.2

### 3-Hydroxy-2-(4-pyridinyl)propénoate de méthyle (III).

On mélange 9 g de 2-(4-pyridinyl)acétate d'éthyle, 1,35 g de NaH à 95 % dans 90 ml de toluène. On chauffe 30 minutes à 100°C puis on refroidit et on ajoute 8,8 ml de formiate d'éthyle. On laisse sous agitation 1 heure à TA et 45 minutes à 100°C. Le milieu réactionnel est refroidi puis on ajoute 25,5 ml d'HCl 2N. On essore puis on reprend le précipité formé par AcOEt puis par de l'éther pour obtenir 5,65 g du composé attendu.

RMN (200 MHz DMSO) : 1,1 ppm : t : 3H ; 4 ppm : q : 2H ; 8 ppm : d : 2H ; 8,6 ppm : d : 2H ; 9,7 ppm : s : 1H.

En opérant comme décrit dans la Préparation 2.1, on a préparé les intermédiaires rassemblés dans le tableau ci-après : (Me représente méthyle)

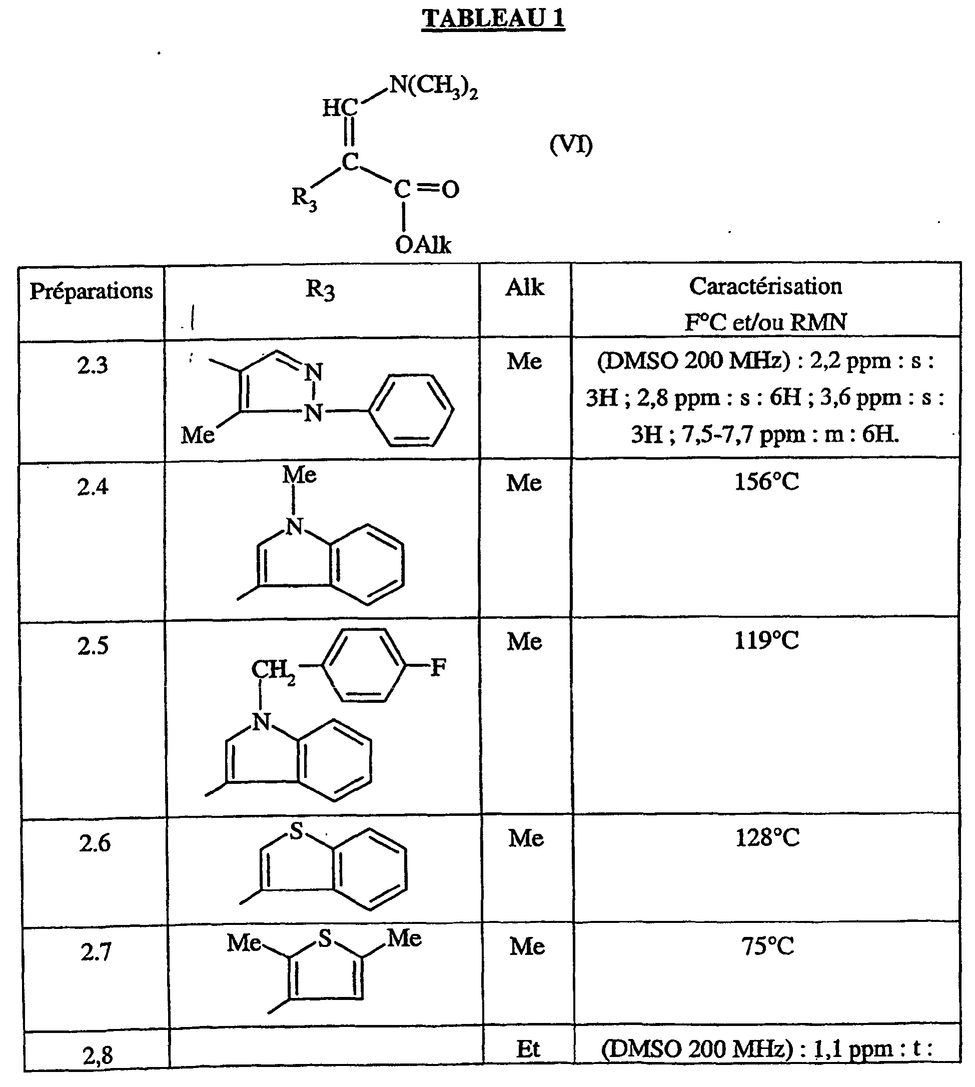

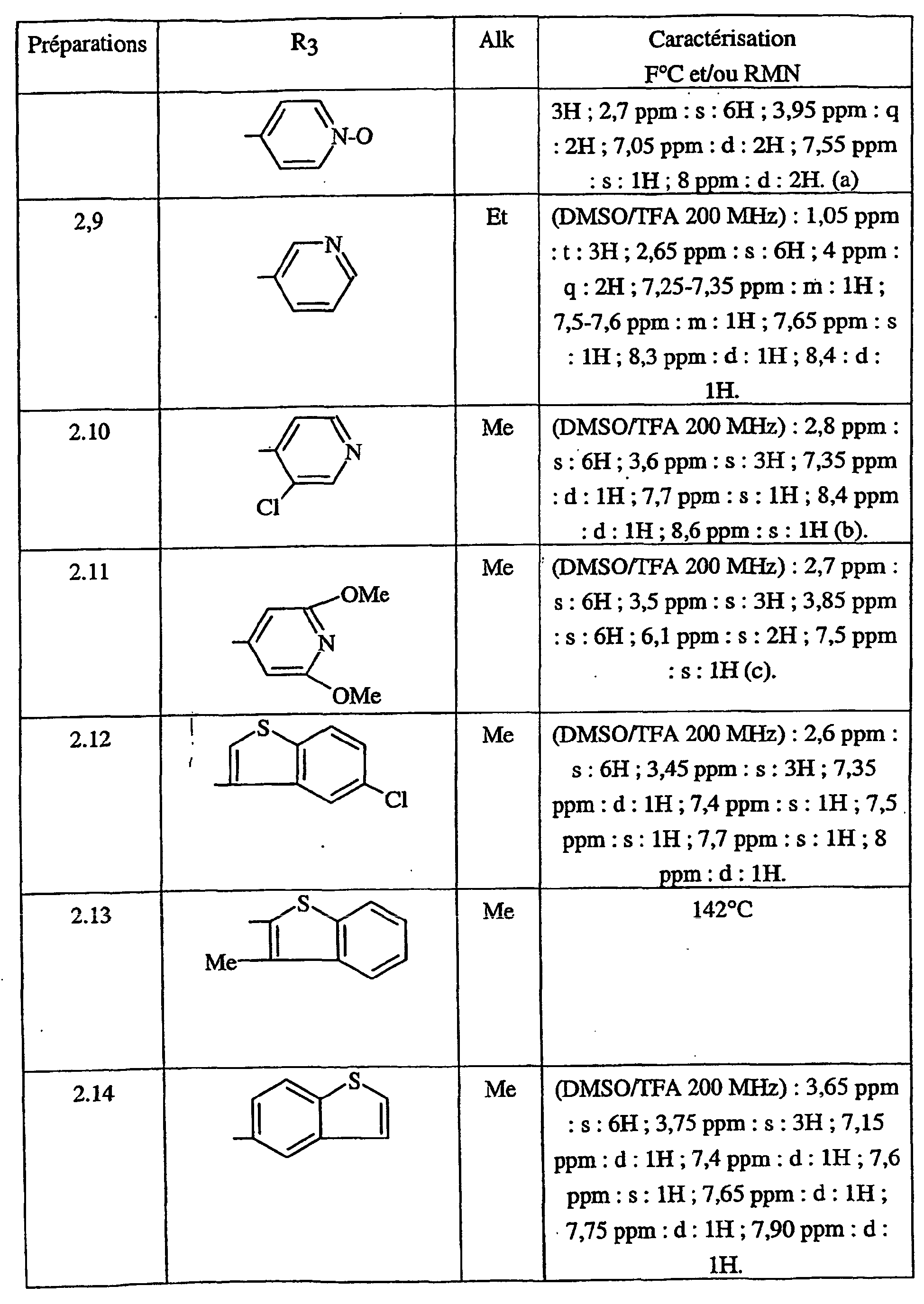

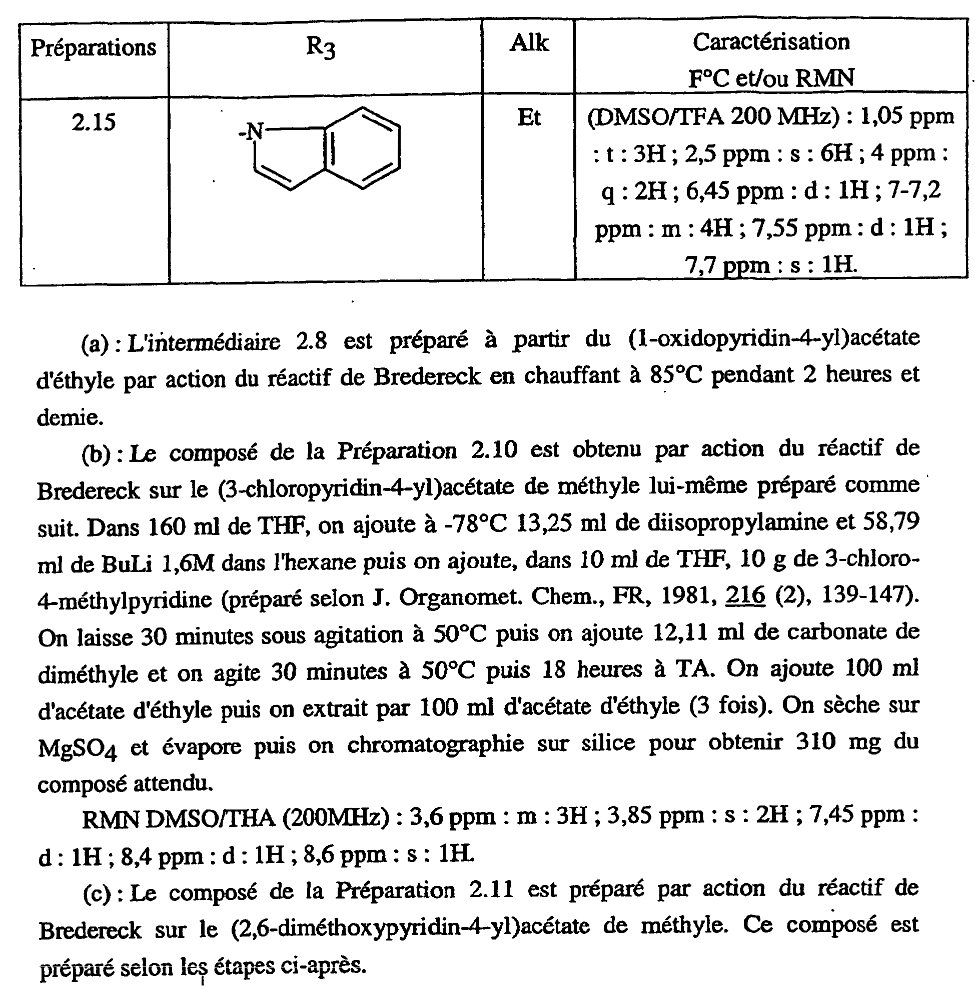

### A) 4-Bromométhyl-2,6-diméthoxypyridine.

On met en solution dans 20 ml de CH₂Cl₂ 1 g de (2,6-diméthoxypyridin-4-yl) méthanol préparé selon J. Heterocl. Chem., 1974, 11, 251, on ajoute 0,84 ml de PBr₃ puis on laisse 10 heures sous agitation à TA. On ajoute du pentane, le précipité formé est essoré, lavé au pentane, puis séché pour obtenir 850 mg du composé attendu.

RMN DMSO (200 MHz) : 3,75 ppm : s : 6H ; 4,45 ppm : s : 2H ; 6,35 ppm : s : 2H.

### B) 4-Cyanométhyl-2,6-diméthoxypyridine.

On mélange 4,3 g du composé de l'étape précédente avec 40 ml d'EtOH et 2,33 g de KCN, on chauffe 2 heures à reflux puis on évapore le solvant. On reprend le résidu par CH₂Cl₂ puis on lave à l'eau, on sèche et évapore. Le solide obtenu est lavé au pentane et on obtient 1,8 g du composé atttendu.

RMN DMSO (200 MHz) : 3,9 ppm : s : 6H ; 4,1 ppm : s : 2H ; 6,4 ppm : s : 2H.

### C) Acide 2,6-diméthoxypyridine-4-acétique.

On place 1,62 g du composé de l'étape précédente dans 50 ml d'EtOH et on ajoute 2,96 ml de NaOH 10 N puis on chauffe à reflux pendant 3 heures. On évapore à sec, reprend par le minimum d'eau puis on porte à pH = 6 par addition DTICI concentré. On extrait par AcOEt, sèche et évapore pour obtenir 1,5 g du composé attendu.

RMN DMSO (200 MHz) : 3,5 ppm : s : 2H ; 3,8 ppm : s : 6H ; 6,25 ppm : s : 2H ; 12,50 ppm : se : 1H.

### D) (2,6-Diméthoxypyridin-4-yl)acetate de méthyle.

On place 1,7 g du composé de l'étape précédente dans 15 ml de MeOH, on refroidit à +4°C puis on ajoute goutte à goutte 2,5 ml de chlorure de thionyle. On laisse 1 heure sous agitation à TA, on évapore à sec, on reprend par MeoH puis on évapore à nouveau et sèche pour obtenir 1 g du composé attendu.

En opérant comme décrit à la Préparation 2.2, on a préparé l'intermédiaire suivant

### Préparation 2.16

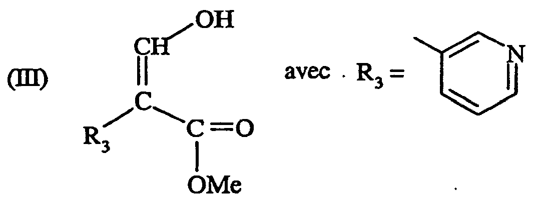

F = 119°C.

### Exemple 1 : composé 3

### Chlorhydrate de 1,6,9-triméthyl-3-(2-pyridinyl)-1,9-dihydro-2H-pyrido[2,3-b] indol-2-one.

On mélange 1 g du composé de la Préparation 1.1 et 1,25 g du composé de la Préparation 2.1 dans 5 ml d'AcOH et on chauffe à 100°C pendant 18 heures. On jette le milieu réactionnel dans 80 ml d'eau et on amène à pH = 9 par addition d'une solution concentrée de NaOH. La gomme obtenue est dissoute dans 30 ml d'un mélange d'AcOEt et DCM (1/1 ; v/v) puis on lave par une solution saturée de NaCl. On sèche sur Na₂SO₄ et évapore pour obtenir une huile jaune qui est reprise dans l'éther chlorhydrique. Le précipité formé est essoré et séché pour donner 200 mg du composé attendu sous forme de poudre jaune, F > 260°C.

RMN (200 MHz DMSO/TFA) : 2,2 ppm : s : 3H ; 3,9 ppm : s : 6H ; 7,1 ppm : d : 1H ; 7,2 ppm : d : 1H ; 7,6 ppm : m : 2H ; 8,2-8,5 ppm : m : 3H ; 8,9 ppm : s : 1H.

### Exemple 2 : composé 2

### Chlorhydrate de 1,6,9-triméthyl-3-(4-pyridinyl)-1,9-dihydro-2H-pyrido[2,3-b] indol-2-one.

On mélange 1 g du composé de la Préparation 1.1 et 0,744 g du composé de la Préparation 2.2 dans 10 ml de pyridine. On chauffe à 100°C jusqu'à la disparition en CCM du composé de la Préparation 2.2 puis on évapore la pyridine et on reprend le résidu par un mélange AcOEt/H₂O (1/1 ; v/v). On sèche sur Na₂SO₄ puis on évapore la phase organique ; le résidu est repris dans AcOEt et on forme un précipité par addition d'éther chlorhydrique. Le précipité est essoré et séché pour donner 180 mg du composé attendu sous forme de poudre jaune, F > 250°C.

RMN (200 MHz DMSO) : 2,6 ppm : s : 3H ; 4 ppm : s : 3H ; 4,1 ppm : s : 3H ; 7,2 ppm : d : 1H ; 7,5 ppm : d : 1H ; 7,9 ppm : s : 1H ; 8,8 ppm : s : 4H ; 9,2 ppm : s : 1H.

### Exemple 3 : composé 4

Chlorhydrate de 1,6-diméthyl-3-pyridin-4-yl-1,9-dihydro-2H-pyrido[2,3-b]indol-2-one.

On dissout 1,5 g du composé de la Préparation 1.2 et 2 g du composé de la Préparation 2.2 dans 20 ml d'AcOH. On chauffe 18 heures à 100°C puis on jette le milieu réactionnel dans 100 ml d'eau. On extrait par Et₂O puis on amène la phase aqueuse à pH = 10 par addition de NaOH concentré. On extrait plusieurs fois par un mélange AcOEt/CH₂Cl₂ (1/1 ; v/v). La phase aqueuse est lavée à l'eau, séchée puis évaporée. Le résidu est dissous dans un minimum de MeOH puis on précipite par de l'éther chlorhydrique. On essore, lave par Et₂O puis sèche pour obtenir 0,680 g du composé attendu.

### Exemple 4 : composé 10

### 3-(1-benzothièn-3-yl)-1,6-diméthyl-1,9-dihydro-2H-pyrido[2,3-b] indol-2-one.

On dissout 2 g du composé de la Préparation 1.2 dans 10 ml d'AcOH et on chauffe à 100°C puis on ajoute 2,66 g du composé de la Préparation 2.6 dans 10 ml d'AcOH et on chauffe à 100°C pendant 2 heures. On refroidit puis on essore le précipité qui est ensuite lavé 2 fois par Et₂O puis séché pour donner 2,47 g du composé attendu.

### Exemple 5 : composé 9

### 3-(1-benzothièn-3-yl)-1,6,9-triméthyl-1,9-dihydro-2H-pyrido[2,3-b] indol-2-one.

On dissout 2,46 g du composé de l'exemple précédent dans 25 ml de DMSO et on ajoute 2,7 g de K₂CO₃ et 5 ml d'iodure de méthyle puis on laisse sous agitation 18 heures à 40°C. Le milieu réactionnel est essoré puis on jette le filtrat dans 300 ml d'eau. On essore de nouveau et on lave le solide 3 fois à l'eau. On sèche, lave 3 fois à l'éther et sèche à nouveau pour obtenir 2,44 g du composé attendu.

### Exemple 6 : composé 13

### 1,6,9-Triméthyl-3-(1-oxidopyridin-4-yl)-2,9-dihydro-1H-pyrido[2,3-b]indol-2-one.

On mélange 300 mg du composé 2 et 30 ml de MeOH puis on ajoute à 0°C 373 mg d'acide m-chloroperbenzoïque dans 5 ml de CH₂Cl₂ et on agite 3 heures à TA. On évapore, reprend par une solution de bisulfite de sodium puis extrait au CH₂Cl₂. On sèche, évapore, puis on reprend par Et₂O, on essore et on sèche à nouveau pour obtenir 235 mg du composé attendu.

Le tableau qui suit illustre les propriétés physiques de quelques composés selon l'invention. Dans ce tableau Me représente méthyle.

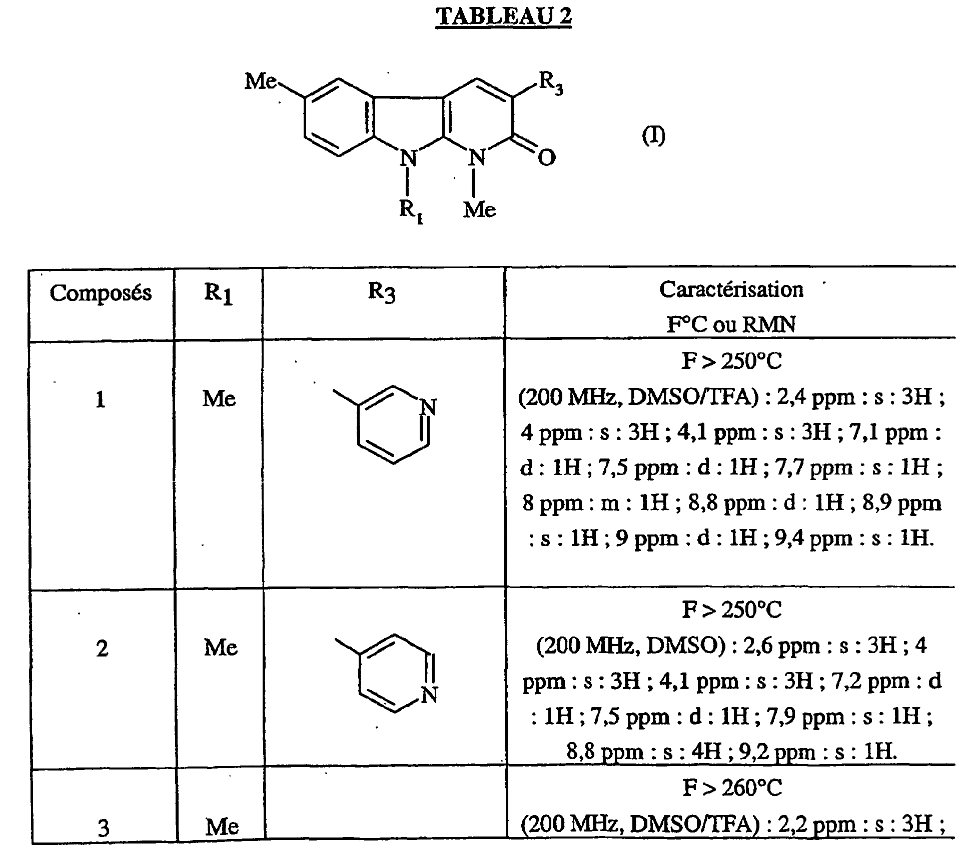

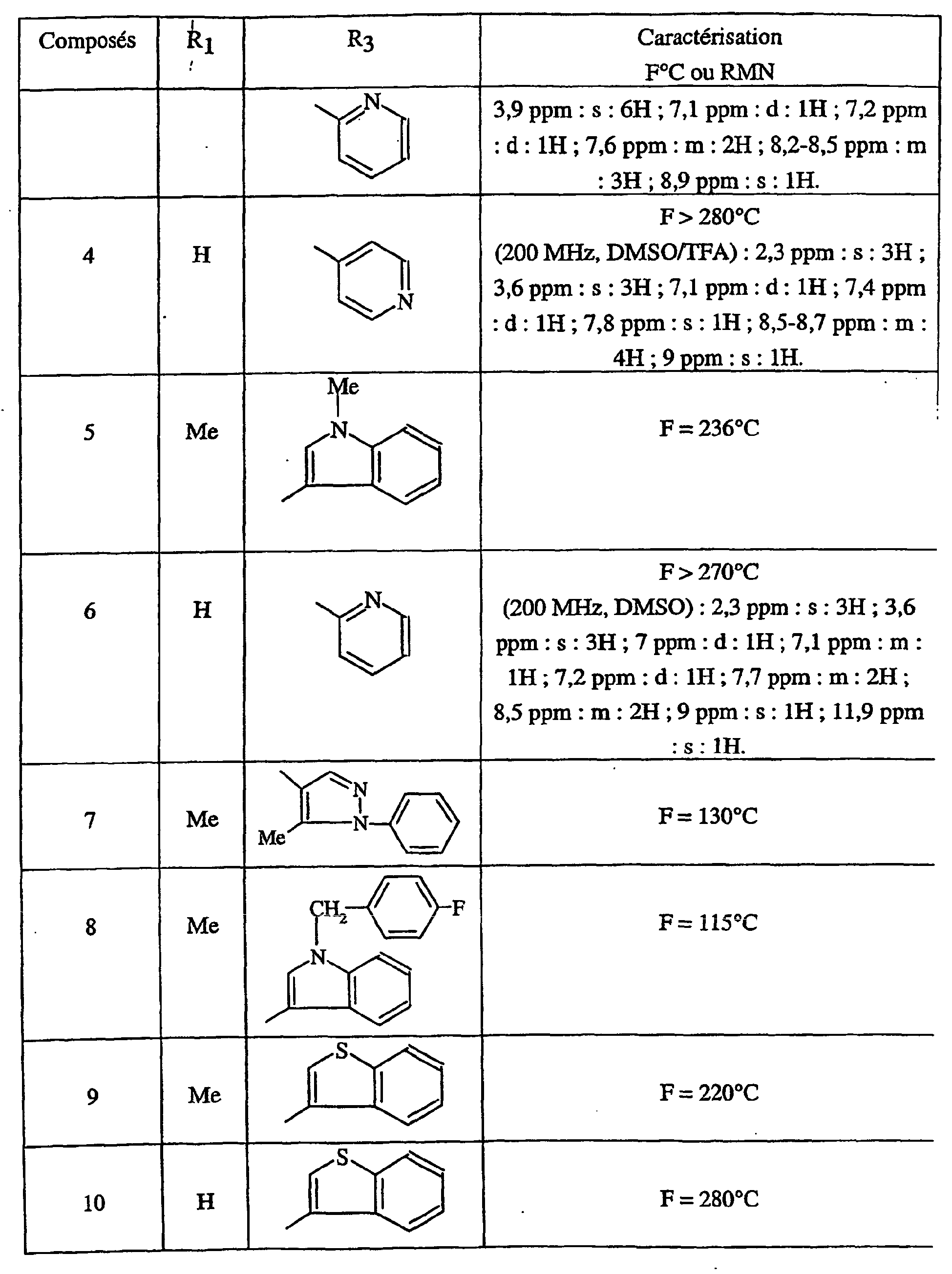

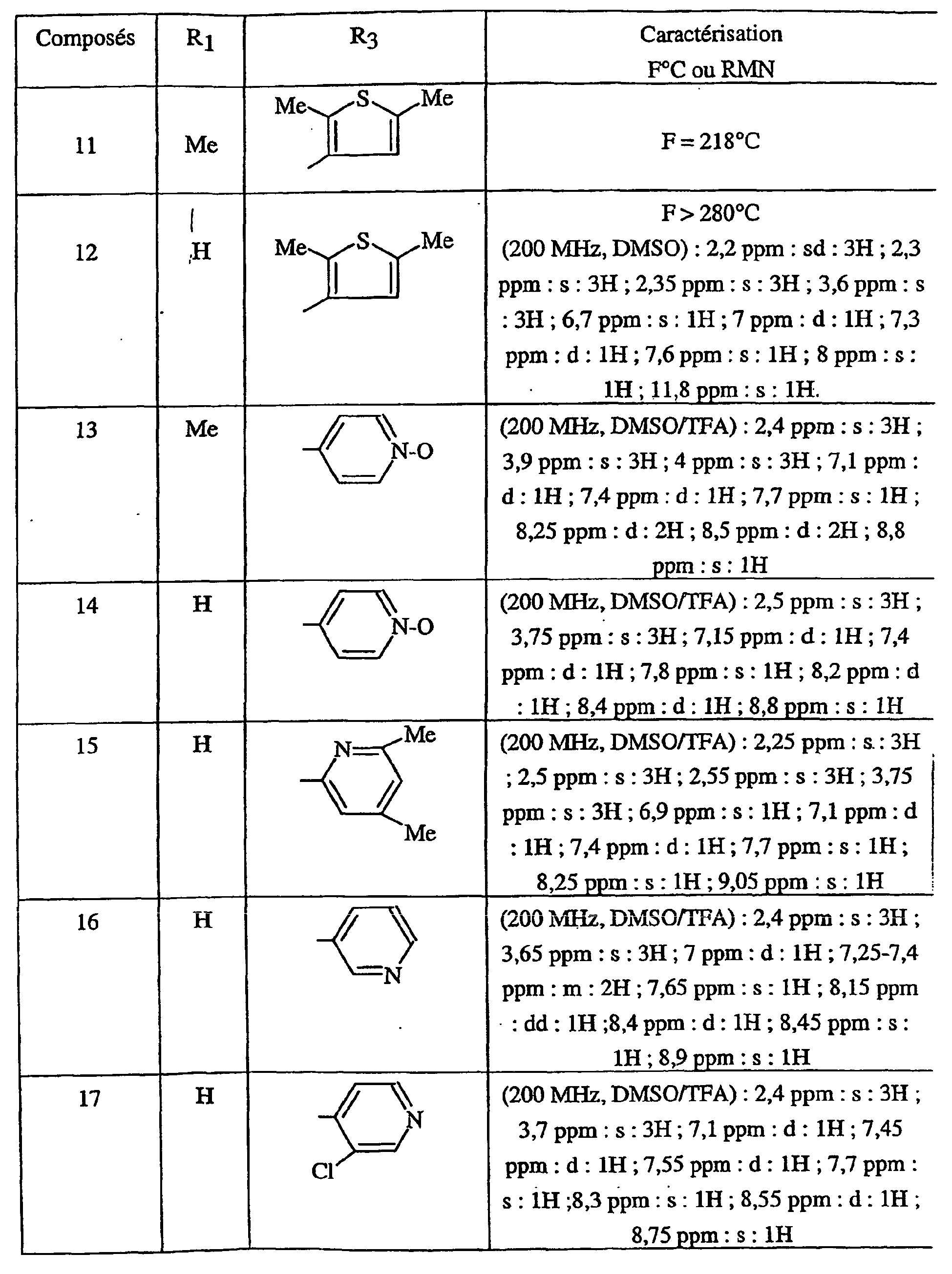

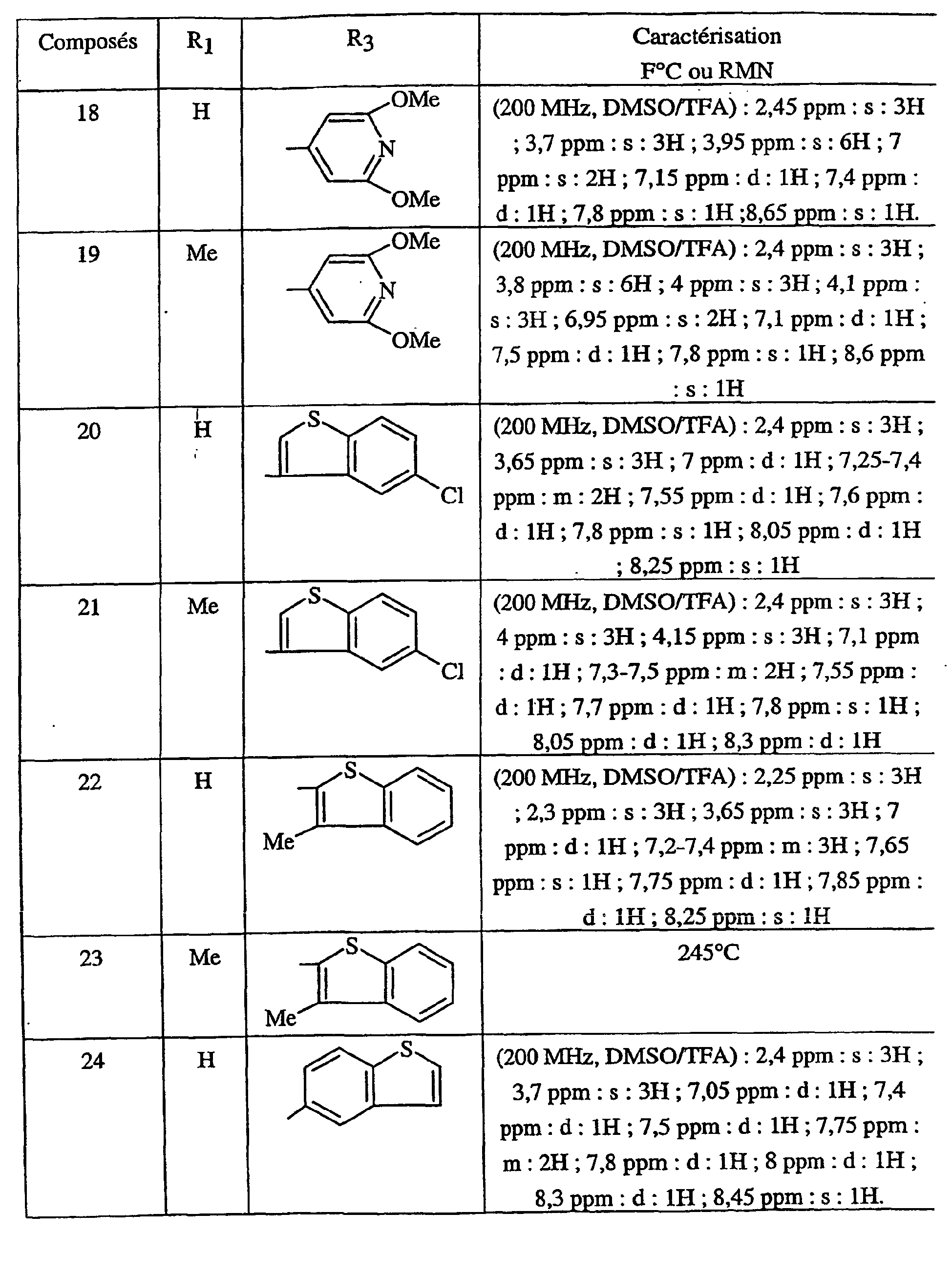

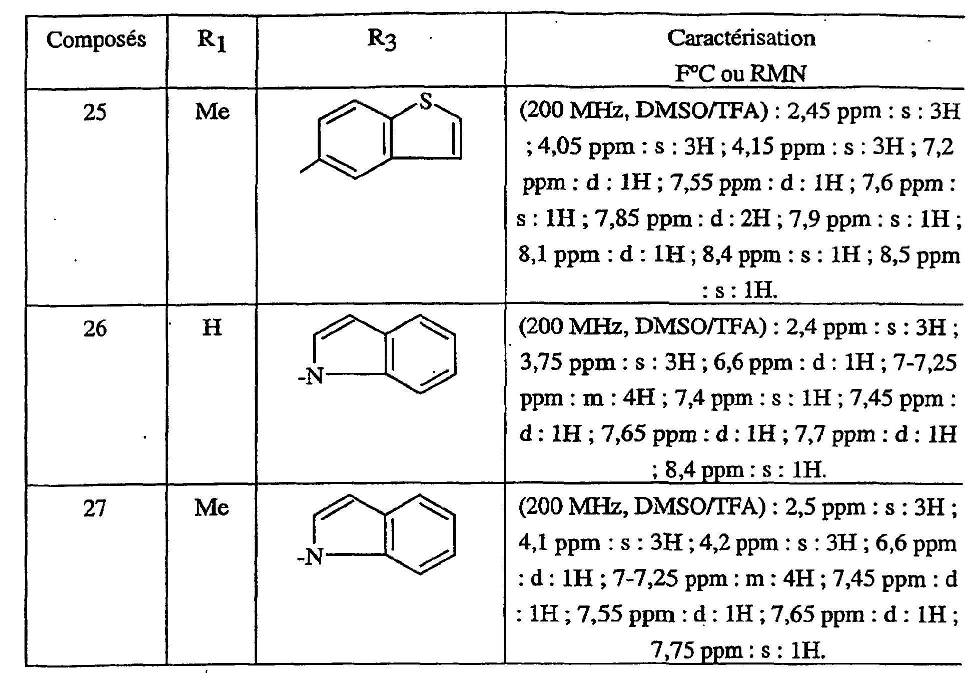

Les composés de formule (I) selon la présente invention ont été testés *in vitro* sur une lignée cellulaire humaine de cancer de sein : la lignée MDA-MB-231 disponible auprès de l'American Type Culture Collection (référence HTB26).

L'évaluation de l'effet antiprolifératif est effectuée selon J.M. Derocq et al., FEBS Letters, 1998, 425, 419-425 : on mesure le taux d'incorporation de la [3H]thymidine dans l'ADN des cellules traitées, après 96 heures d'incubation d'un composé de formule (I). La concentration inhibitrice 50 (CI₅₀) est définie comme la concentration qui inhibe la prolifération cellulaire de 50 %.

Les composés selon l'invention présentent une CI₅₀ généralement inférieure à 10 µM sur la lignée MDA-MB-231.

Les composés de formule (I) ont également été testés sur une autre lignée cellulaire humaine de cancer du sein, dite lignée multi-résistante MDR, (de l'anglais multi-drug-resistant) et appelée MDA-A₁. Cette lignée est décrite par E. Collomb, C. Dussert et P.M. Martin dans Cytometry, 1991, 12(1), 15-25.

Le terme "multi-résistant" qui qualifie cette lignée, signifie que ladite lignée est peu sensible d'une manière générale aux drogues de chimiothérapie communément utilisée et en particulier aux antimitotiques d'origine naturelle tels que le paclitaxel, la vincristine, la vinblastine.

Les composés selon l'invention présentent une CI₅₀ généralement inférieure à 10 µM sur la lignée multi-résistante MDA-A₁.

Les composés selon l'invention ont également été testés *in vivo* dans des modèles murins de xénogreffes de tumeurs humaines selon les méthodes décrites dans la littérature : Mooberry SL et al., Int. J. Cancer, 2003, 104 (4), 512-521 ; Polin L et al., Invest. New Drugs, 2002, 20 (1), 13-22 ; Corbett TH et al., Invest. New Drugs, 1999, 17 (1), 17-27. Des fragments de tumeurs humaines de 2 à 3 mm de diamètre sont implantés de façon sous-cutanée chez des souris SCID (de l'anglais Severe Combined Immunodeficiency) de souche Balb/C (Iffa-Credo, Lyon, France). Lorsque ces tumeurs atteignent un poids de 50-60 mg, les composés sont administrés par voie orale ou intraveineuse tous les jours ou tous les deux jours pendant toute la durée de l'expérience (20 à 40 jours) à des doses variant de 10 à 300 mg/kg par administration. Le poids des tumeurs est estimé selon la formule : P (poids de la tumeur en mg) = (axb²)/2, où a et b représentent respectivement la longueur et la largeur en mm de l'implant tumoral. La mesure de a et de b est réalisée à l'aide d'un pied à coulisse. L'efficacité antitumorale est évaluée en comparant le poids moyen des tumeurs dans le groupe des animaux traités avec le composé à l'étude (T) à celui des animaux du groupe contrôle auxquels on a administré uniquement le solvant du composé (C). Cette mesure, exprimée en % du rapport T/C, est réalisée lorsque C atteint approximativement 1000 mg. Les composés selon l'invention ont démontré une activité antitumorale *in vivo* (rapport T/C inférieur à 100 %), certains de façon très significative avec un rapport T/C inférieur ou égal à 42 %.

Ainsi, selon la présente invention, il apparaît que les composés de formule (I) inhibent la prolifération des cellules tumorales y compris celle des cellules présentant une multi-résistance. Il apparaît donc que les composés selon l'invention ont une activité anticancéreuse.

Ainsi selon un autre de ses aspects, l'invention a pour objet des médicaments qui comprennent un composé de formule (I), ou un sel d'addition de ce dernier à un acide pharmaceutiquement acceptable ou encore un hydrate ou un solvat du composé de formule (I).

Ces médicaments trouvent leur emploi en thérapeutique, notamment dans le traitement ou la prévention des maladies causées ou exacerbées par la prolifération des cellules tumorales.

Comme inhibiteur de la prolifération des cellules tumorales, ces composés sont utiles dans le traitement des tumeurs solides à la fois primaires et métastasiques, des carcinomes et cancers, en particulier : cancer du sein ; cancer du poumon ; cancer de l'intestin grêle, cancer du colon et du rectum ; cancer des voies respiratoires, de l'oropharynx et de l'hypopharynx ; cancer de l'oesophage; cancer du foie, cancer de l'estomac, cancer des canaux biliaires, cancer de la vésicule biliaire, cancer du pancréas; cancers des voies urinaires y compris rein, urothelium et vessie; cancers du tractus génital féminin y compris cancer de l'utérus, du col de l'utérus, des ovaires, chloriocarcinome et trophoblastome; cancers du tractus génital masculin y compris cancer de la prostate, des vésicules séminales, des testicules, tumeurs des cellules germinales; cancers des glandes endocrines y compris cancer de la thyroïde, de l'hypophyse, des glandes surrénales ; cancers de là peau y compris hémangiomes, mélanomes, sarcomes, incluant le sarcome de Kaposi ; tumeurs du cerveau, des nerfs, des yeux, des méninges, incluant astrocytomes, gliomes, glioblastomes, rétinoblastomes, neurinomes, neuroblastomes, schwannomes, méningiomes ; tumeurs solides venant de tumeurs malignes hématopoïétiques incluant leucémies, chloromes, plasmacytomes, mycosis fongoïde, lymphome ou leucémie des cellules T, lymphome non hodgkinien, hémopathies malignes, myélomes.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, un hydrate ou solvat dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

Les composés de formule (I) ci-dessus peuvent être utilisés à des doses journalières de 0,002 à 2000 mg par kilogramme de poids corporel du mammifère à traiter, de préférence à des doses journalières de 0,1 à 300 mg/kg. Chez l'être humain, la dose peut varier de préférence de 0,02 à 10000 mg par jour, plus particulièrement de 1 à 3000 mg selon l'âge du sujet à traiter ou le type de traitement : prophylactique ou curatif.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, concerne également une méthode de traitement des pathologies ci-dessus indiquées qui comprend l'administration, à un patient, d'une dose efficace d'un composé selon l'invention, ou un de ses sels pharmaceutiquement acceptables ou hydrates ou solvats.

Selon la présente invention, le ou les composés de formule (I) peuvent être administrés en association avec un (ou plusieurs) principe(s) actif(s) anticancéreux, en particulier des composés antitumoraux tels que les agents alkylants tels que les alkylsulfonates (busulfan), la dacarbazine, la procarbazine, les moutardes azotées (chlorméthine, melphalan, chlorambucil), cyclophosphamide, ifosfamide; les nitrosourées tels que la carmustine, la lomustine, la sémustine, la streptozocine; les alcaloïdes antinéoplasiques tels que la vincristine, la vinblastine ; les taxanes tel que le paclitaxel ou le taxotère ; les antibiotiques antinéoplasiques tels que l'actinomycine; les agents intercalants, les antimétabolites antinéoplasiques, les antagonistes des folates, le méthotrexate ; les inhibiteurs de la synthèse des purines, les analogues de la vurine tels que mercaptopurine, 6-thioguanine; les inhibiteurs de la synthèse des pyrimidines, les inhibiteurs d'aromatase, la capécitabine, les analogues de la pyrimidine tels que fluorouracil, gemcitabine, cytarabine et cytosine arabinoside ; le bréquinar ; les inhibiteurs de topoisomérases tels que la camptothécine ou l'étoposide ; les agonistes et antagonistes hormonaux anticancéreux incluant le tamoxifène ; les inhibiteurs de kinase, l'imatinib ; les inhibiteurs de facteurs de croissance ; les antiinflammatoires tels que le pentosane polysulfate, les corticostéroïdes, la prednisone, la dexamethasone ; les antitopoisomérases tels que l'étoposide, les antracyclines incluant la doxorubicine, la bléomycine, la mitomycine et la méthramycine ; les complexes métalliques anticancéreux, les complexes du platine, le cisplatine, le carboplatine, foxaliplatine ; l'interféron alpha, le triphénylthiophosphoramide, l'altrétamine ; les agents antiangiogéniques ; la thalidomide ; les adjuvants d'immunothérapie ; les vaccins.

La présente invention, selon un autre de ses aspects, concerne également une méthode de traitement des pathologies ci-dessus indiquées qui comprend l'administration, à un patient, d'une dose efficace d'un composé selon l'invention, ou un de ses sels pharmaceutiquement acceptables ou hydrates ou solvats.

## Revendications

1. Composé répondant à la formule : 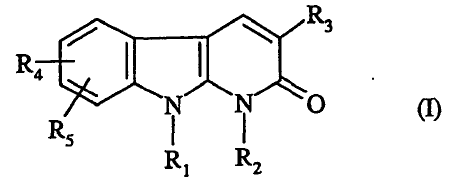 dans laquelle :
- R₁ représente un atome d'hydrogène ou un groupe (C₁-C₄)alkyle ;
- R₂ représente un atome d'hydrogène ou un groupe (C₁-C₄)alkyle ;
- R₃ représente un thiényle mono ou poly substitué par un groupe méthyle ; ou un radical hétérocyclique monocyclique ou bicyclique choisi parmi : un pyridyle, un N-oxydopyridyle, un pyrazole, un (N-phényl)pyrazole, un (N-halogénophényl)pyrazole, un furyle, un indolyle, un (N-benzyl)indolyle, un (N-halogénobenzyl)indolyle, un benzothiényle, un benzofuryle, lesdits radicaux étant non substitués ou substitués une ou plusieurs fois par un atome d'halogène ou un groupe méthyle ou méthoxy ;
- R₄ et R₅ sont identiques ou différents et représentent chacun indépendamment un atome d'hydrogène ou d'halogène ou un groupe hydroxyle, un hydroxyméthyle, (C₁-C₄)alkyle, trifluorométhyle, (C₁-C₄)alcoxy, (C₁₋C₄)alcoxycarbonyle, un cyano ;
ainsi qu'à l'état de base ou de sel d'addition à un acide, ainsi qu'à l'état d'hydrate ou de solvat.

2. Composé de formule (I) selon la revendication 1 **caractérisé en ce que** :
- R₁ représente un atome d'hydrogène ou un groupe méthyle ;
- et/ou R₂ représente un groupe méthyle ;
- et/ou R₃ représente un radical hétérocyclique choisi parmi un N-oxidopyridyle, un pyridyle, un benzothiényle, lesdits radicaux étant non substitués ou substitués une ou plusieurs fois par un atome d'halogène ou un groupe méthyle ou méthoxy ; et/ou R₄ représente un groupe méthyle ;
et/ou R₅ représente un atome d'hydrogène ou un groupe méthyle ;
à l'état de base ou de sel d'addition à un acide, ainsi qu'à l'état d'hydrate ou de solvat.

3. Composés selon la revendication 1 choisi parmi :
1,6-Diméthyl-3-pyridin-4-yl-1,9-dihydro-2*H*-pyrido[2,3-b]indol-2-one ;
. 1,6,9-Triméthyl-3-pyridin-4-yl-1,9-dihydro-2*H*-pyrido[2,3-b]indol-2-one ;
. 1,6-Diméthyl-3-(1-oxidopyridin-4-yl)-1,9-dihydro-2*H*-pyrido[2,3-b] indol-2-one;
. 3-(4,6-Diméthylpyridin-2-yl)-1,6-diméthyl-1,9-dihydro-2*H*-pyrido[2,3-b] indol-2-one ;
. 3-(1-Benzothien-5-yl)-1,6-diméthyl-1,9-dihydro-2*H*-pyrido[2,3-b] indol-2-one ;
. 3-(5-Chloro-1-benzothién-3-yl)-1,6,9-triméthyl-1,9-dihydro-2*H*-pyrido[2,3-b]indol-2-one ;
. 3-(1*H*-Indol-1-yl)-1,6-diméthyl-1,9-dihydro-2*H*-pyrido[2,3-b] indol-2-one ;
. 3-(3-Chloropyridin-4-yl)-1,6-diméthyl-1,9-dihydro-2*H*-pyrido[2,3-b] indol-2-one ;
. 3-(2,6-Diméthoxypyridin-4-yl)-1,6-diméthyl-1,9-dihydro-2*H*-pyrido[2,3-b] indol-2-one ;
. 3-(2,6-Diméthoxypyridin-4-yl)-1,6,9-triméthyl-1,9-dihydro-2*H*-pyrido[2,3-b] indol-2-one ;
à l'état de base ou de sel d'addition à un acide, ainsi qu'à l'état d'hydrate ou de solvat.

4. Procédé de préparation d'un composé de formule (I) selon l'une quelconque des revendications 1 à 3, **caractérisé en ce que** :
on fait réagir un 2-aminoindole de formule : 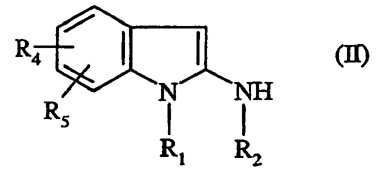
dans laquelle R₁, R₂, R₄ et R₅ sont tels que définis pour un composé de formule (1) à la revendication 1 avec un ester de formule : 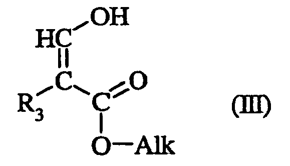 dans laquelle **R**_{**3**} est tel que défini pour un composé de formule (I) à la revendication 1 et Alk représente un alkyle en C₁-C₄.

5. Procédé de préparation d'un composé de formule (I) selon l'une quelconque des revendications 1 à 3, **caractérisé en ce que** :
on fait réagir un aminoindole de formule : 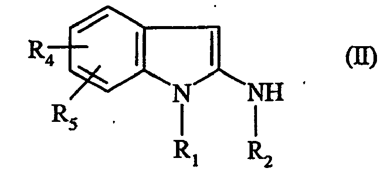
dans laquelle R₁, R₂, R₄ et R₅ sont tels que définis pour un composé de formule (I) à la revendication 1 avec un ester de formule : 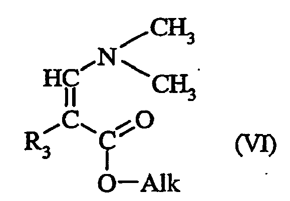 dans laquelle R₃ est tel que défini pour un composé de formule (I) à la revendication 1 et Alk représente un alkyle en C₁-C₄.

6. Médicament **caractérisé en ce qu'**il contient un composé selon l'une quelconque des revendications 1 à 3, ou un sel d'addition de ce composé à un acide pharmaceutiquement acceptable, ou encore un hydrate ou un solvant du composé de formule (I).

7. Composition pharmaceutique **caractérisé en ce qu'**elle comprend un composé de formule (I) selon l'une quelconque des revendications 1 à 3, ou un sel pharmaceutiquement acceptable ou un hydrate ou un solvat.

8. Utilisation d'un composé de formule (I) selon l'une quelconque des revendications 1 à 3, pour la préparation d'un médicament destiné au traitement et à la prévention des maladies causées ou exacerbées par la prolifération des cellules tumorales.

## Patentansprüche

1. Verbindung, die der Formel: 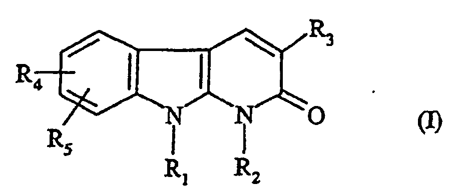 entspricht, worin:
- R₁ ein Wasserstoffatom oder eine (C₁-C₄)Alkylgruppe darstellt;
- R₂ ein Wasserstoffatom oder eine (C₁-C₄)Alkylgruppe darstellt;
- R₃ ein mit einer Methylgruppe mono- oder polysubstituiertes Thienyl darstellt; oder einen monocyclischen oder bicyclischen heterocyclischen Rest, der ausgewählt ist unter: einem Pyridyl, einem N-Oxidopyridyl, einem Pyrazol, einem (N-Phenyl)pyrazol, einem (N-Halogenphenyl)pyrazol, einem Furyl, einem Indolyl, einem (N-Benzyl)indolyl, einem (N-Halogenbenzyl)indolyl, einem Benzothienyl, einem Benzofuryl, wobei besagte Reste nicht substituiert sind oder ein- oder mehrfach mit einem Halogenatom oder einer Methyl- oder Methoxygruppe substituiert sind;
- R₄ und R₅ gleich oder verschieden sind und jeweils unabhängig voneinander ein Wasserstoff- oder Halogenatom oder eine Hydroxylgruppe, ein Hydroxymethyl, (C₁-C₄)Alkyl, Trifluormethyl, (C₁-C₄)Alkoxy, (C₁-C₄)Alkoxycarbonyl, ein Cyano darstellen;
sowohl in Form von Base oder Additionssalz mit einer Säure als auch in Form von Hydrat oder Solvat.

2. Verbindung der Formel (I) gemäß Anspruch 1, **dadurch gekennzeichnet, dass**:
- R₁ ein Wasserstoffatom oder eine Methylgruppe darstellt;
- und/oder R₂ eine Methylgruppe darstellt;
- und/oder R₃ einen heterocyclischen Rest darstellt, der ausgewählt ist unter einem N-Oxidopyridyl, einem Pyridyl, einem Benzothienyl, wobei besagte Reste nicht substituiert sind oder ein- oder mehrfach mit einem Halogenatom oder einer Methyl- oder Methoxygruppe substituiert sind;
- und/oder R₄ eine Methylgruppe darstellt;
- und/oder R₅ ein Wasserstoffatom oder eine Methylgruppe darstellt;
in Form von Base oder Additionssalz mit einer Säure sowie in Form von Hydrat oder Solvat.

3. Verbindungen gemäß Anspruch 1, ausgewählt unter:
- 1,6-Dimethyl-3-pyridin-4-yl-1,9-dihydro-2*H*-pyrido[2,3-b]indol-2-on;
- 1,6,9-Trimethyl-3-pyridin-4-yl-1,9-dihydro-2*H*-pyrido[2,3-b]indol-2-on;
- 1,6-Dimethyl-3-(1-oxidopyridin-4-yl)-1,9-dihydro-2*H*-pyrido[2,3-b]indol-2-on;
- 3-(4,6-Dimethylpyridin-2-yl)-1,6-dimethyl-1,9-dihydro-2*H*-pyrido[2,3-b]indol-2-on;
- 3-(1-Benzothien-5-yl)-1,6-dimethyl-1,9-dihydro-2*H*-pyrido[2,3-b]indol-2-on;
- 3-(5-Chlor-1-benzothien-3-yl)-1,6,9-trimethyl-1,9-dihydro-2*H*-pyrido[2,3-b]indol-2-on;
- 3-(1*H*-Indol-1-yl)-1,6-dimethyl-1,9-dihydro-2*H*-pyrido[2,3-b]indol-2-on;
- 3-(3-Chlorpyridin-4-yl)-1,6-dimethyl-1,9-dihydro-2*H*-pyrido[2,3-b]indol-2-on;
- 3-(2,6-Dimethoxypyridin-4-yl)-1,6-dimethyl-1,9-dihydro-2*H*-pyrido[2,3-b]indol-2-on;
- 3-(2,6-Dimethoxypyridin-4-yl)-1,6,9-trimethyl-1,9-dihydro-2*H*-pyrido[2,3-b]indol-2-on;
in Form von Base oder Additionssalz mit einer Säure sowie in Form von Hydrat oder Solvat.

4. Verfahren zur Herstellung einer Verbindung der Formel (I) gemäß einem der Ansprüche 1 bis 3, **dadurch gekennzeichnet, dass**:
man ein 2-Aminoindol der Formel: 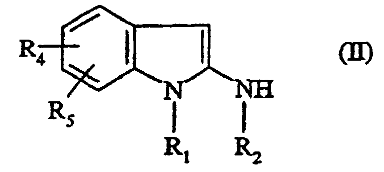
worin R₁, R₂, R₄ und R₅ wie für eine Verbindung der Formel (I) in Anspruch 1 definiert sind, mit einem Ester der Formel: 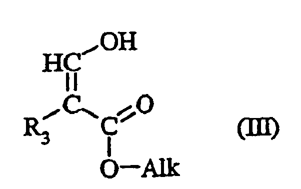 worin R₃ wie für eine Verbindung der Formel (I) in Anspruch 1 definiert ist und Alk ein C₁-C₄-Alkyl darstellt, umsetzt.

5. Verfahren zur Herstellung einer Verbindung der Formel (I) gemäß einem der Ansprüche 1 bis 3, **dadurch gekennzeichnet, dass**:
man ein Aminoindol der Formel: 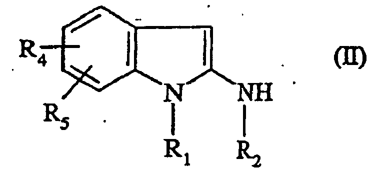
worin R₁, R₂, R₄ und R₅ wie für eine Verbindung der Formel (I) in Anspruch 1 definiert sind, mit einem Ester der Formel: 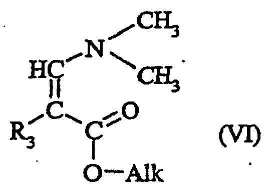 worin R₃ wie für eine Verbindung der Formel (I) in Anspruch 1 definiert ist und Alk ein C₁-C₄-Alkyl darstellt, umsetzt.

6. Arzneimittel, **dadurch gekennzeichnet, dass** es eine Verbindung gemäß einem der Ansprüche 1 bis 3 oder ein Additionssalz dieser Verbindung mit einer pharmazeutisch annehmbaren Säure oder auch ein Hydrat oder ein Solvat der Verbindung der Formel (I) enthält.

7. Pharmazeutische Zusammensetzung, **dadurch gekennzeichnet, dass** sie eine Verbindung der Formel (I) gemäß einem der Ansprüche 1 bis 3 oder ein pharmazeutisch annehmbares Salz oder ein Hydrat oder ein Solvat umfasst.

8. Verwendung einer Verbindung der Formel (I) gemäß einem der Ansprüche 1 bis 3 zur Herstellung eines Arzneimittels, das für die Behandlung und für die Prophylaxe der Krankheiten bestimmt ist, die durch die Proliferation der Tumorzellen verursacht oder verschlimmert werden.

## Claims

1. Compound corresponding to the formula: 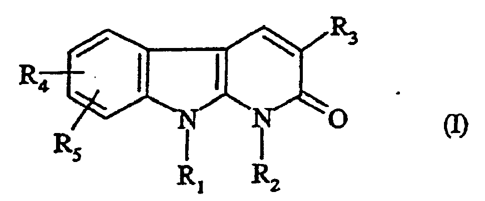 in which:
- R₁ represents a hydrogen atom or a (C₁-C₄)alkyl group;
- R₂ represents a hydrogen atom or a (C₁-C₄)alkyl group;
- R₃ represents a thienyl mono- or polysubstituted by a methyl group; or a monocyclic or bicyclic heterocyclic radical chosen from: a pyridyl, an N-oxidopyridyl, a pyrazolyl, an (N-phenyl)-pyrazolyl, an (N-halophenyl)pyrazolyl, a furyl, an indolyl, an (N-benzyl)indolyl, an (N-halobenzyl)indolyl, a benzothienyl or a benzofuryl, the said radicals being unsubstituted or substituted one or more times by a halogen atom or a methyl or methoxy group;
- R₄ and R₅ are identical or different and each independently represent a hydrogen or halogen atom or a hydroxyl, hydroxymethyl, (C₁-C₄)alkyl, trifluoromethyl, (C₁-C₄)alkoxy, (C₁-C₄)alkoxycarbonyl or cyano group;
in the form of the base or of an addition salt with an acid, and in the hydrate or solvate form.

2. Compound of formula (I) according to Claim 1, **characterized in that**:
- R₁ represents a hydrogen atom or a methyl group;
- and/or R₂ represents a methyl group;
- and/or R₃ represents a heterocyclic radical chosen from an N-oxidopyridyl, a pyridyl or a benzothienyl, the said radicals being unsubstituted or substituted one or more times by a halogen atom or a methyl or methoxy group;
and/or R₄ represents a methyl group;
and/or R₅ represents a hydrogen atom or a methyl group;
in the form of the base or of an addition salt with an acid, and in the hydrate or solvate form.

3. Compounds according to Claim 1, chosen from:
· 1,6-dimethyl-3-(pyridin-4-yl)-1,9-dihydro-2H-pyrido[2,3-b]indol-2-one;
· 1,6,9-trimethyl-3-(pyridin-4-yl)-1,9-dihydro-2H-pyrido[2,3-b]indol-2-one;
· 1,6-dimethyl-3-(1-oxidopyridin-4-yl)-1,9-dihydro-2H-pyrido[2,3-b]indol-2-one;
· 3-(4,6-dimethylpyridin-2-yl)-1,6-dimethyl-1,9-dihydro-2H-pyrido[2,3-b]indol-2-one;
· 3-(1-benzothien-5-yl)-1,6-dimethyl-1,9-dihydro-2H-pyrido [2, 3-b] indol-2-one;
· 3-(5-chloro-1-benzothien-3-yl)-1,6,9-trimethyl-1,9-dihydro-2*H*-pyrido[2,3-b]indol-2-one;
· 3-(1*H*-indol-1-yl)-1,6-dimethyl-1,9-dihydro-2*H-*pyrido[2,3-b]indol-2-one;
· 3-(3-chloropyridin-4-yl)-1,6-dimethyl-1,9-dihydro-2*H*-pyrido[2,3-b]indol-2-one;
· 3-(2,6-dimethoxypyridin-4-yl)-1,6-dimethyl-1,9-dihydro-2*H*-pyrido[2,3-b]indol-2-one;
· 3-(2,6-dimethoxypyridin-4-yl)-1,6,9-trimethyl-1,9-dihydro-2*H*-pyrido[2,3-b]indol-2-one;
in the form of the base or of an addition salt with an acid, and in the hydrate or solvate form.

4. Process for the preparation of a compound of formula (I) according to any one of Claims 1 to 3,
**characterized in that**:
a 2-aminoindole of formula: 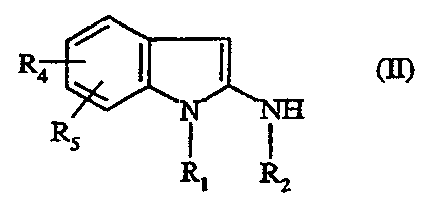
in which R₁, R₂, R₄ and R₅ are as defined for a compound of formula (I) in Claim 1, is reacted with an ester of formula: 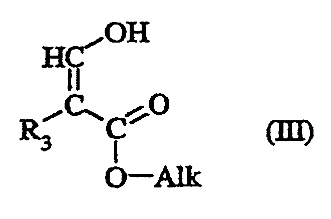 in which R₃ is as defined for a compound of formula (I) in Claim 1 and Alk represents a C₁-C₄ alkyl.

5. Process for the preparation of a compound of formula (I) according to any one of Claims 1 to 3,
**characterized in that**:
an aminoindole of formula: 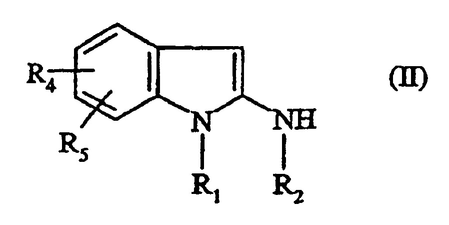
in which R₁, R₂, R₄ and R₅ are as defined for a compound of formula (I) in Claim 1, is reacted with an ester of formula: 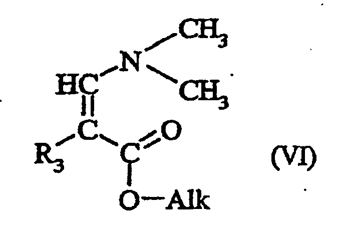 in which R₃ is as defined for a compound of formula (I) in Claim 1 and Alk represents a C₁-C₄ alkyl.

6. Medicament, **characterized in that** it comprises a compound according to any one of Claims 1 to 3, or an addition salt of this compound with a pharmaceutically acceptable acid, or a hydrate or a solvate of the compound of formula (I).

7. Pharmaceutical composition, **characterized in that** it comprises a compound of formula (I) according to any one of Claims 1 to 3, or a pharmaceutically acceptable salt or a hydrate or a solvate.

8. Use of a compound of formula (I) according to any one of Claims 1 to 3 in the preparation of a medicament intended for the treatment and for the prevention of diseases caused or exacerbated by the proliferation of tumour cells.
